# EUROPEAN PATENT SPECIFICATION

(11) **EP 4 112 087 B1**
(45) Date of publication and mention of the grant of the patent: **13.11.2024**
(21) Application number: 20921680.3
(22) Date of filing: 04.08.2020
(51) Int. Cl.: F24F 8/20, F24F 8/10, F24F 8/22, A61L 9/14, A61L 9/20, F24F 11/00, F24F 8/133

(54) **EPIDEMIC PREVENTION CHANNEL AND EPIDEMIC PREVENTION METHOD**
EPIDEMIEVERHINDERUNGSKANAL UND VERFAHREN ZUR EPIDEMIEVERHINDERUNG
CANAL ET PROCÉDÉ DE PRÉVENTION DES ÉPIDÉMIES

(30) Priority: 24.02.2020 CN 202010112703
(43) Date of publication of application: 04.01.2023
(73) Proprietor: Guangdong Provincial Academy of Building Research Group Co., Ltd, Guangzhou, Guangdong 510500 (CN)
(72) Inventor: YU, Peng, Guangzhou, Guangdong 510500 (CN); YANG, Shichao, Guangzhou, Guangdong 510500 (CN); LUO, Yunyou, Guangzhou, Guangdong 510500 (CN); LIANG, Hao, Guangzhou, Guangdong 510500 (CN); DING, Jun, Guangzhou, Guangdong 510500 (CN)
(74) Representative: Becker, Eberhard
(86) International application number: PCT/CN2020/106698
(87) International publication number: WO 2021/169179

(56) References cited:
- EP-A1- 1 736 712
- WO-A1-2010/016082
- WO-A1-2013/006974
- WO-A2-2007/026363
- CN-A- 111 317 839
- CN-U- 203 036 779
- CN-U- 205 664 524
- CN-U- 207 350 579
- CN-U- 208 832 610
- CN-U- 209 944 597
- JP-A- 2006 122 261
- KR-A- 20120 113 324
- KR-B1- 101 973 931
- US-A- 5 692 954
- US-A1- 2016 265 796

## Description

### BACKGROUND

### Technical Field

The disclosure belongs to an epidemic prevention technology, and in particular, the invention relates to an epidemic prevention channel and an epidemic prevention method.

### Description of Related Art

In 2020, the novel coronavirus pneumonia epidemic (named by the WHO as COVID-19) broke out globally. The virus is highly contagious, spreads fast, and greatly affects the social production order, so it will cause immeasurable losses to the global economy. Further, due to the emergency and high transmission rates of COVID-19, it has caused social panic to some extent, and the task of epidemic prevention and control is thereby arduous.

The SARS epidemic that broke out in 2003 caused a total of 5,327 cases and 349 deaths in China, resulting in a direct economic loss of 93.3 billion yuan in China. Besides, winter and spring are the seasons of high incidence of epidemic and infectious diseases in southern China, such as influenza and bird flu. Every year, a large number of people are infected with such epidemics, and production and life are greatly affected.

After the outbreak, an epidemic prevention channel can be set up at the entrance of the building to carry out epidemic prevention and control work. At present, in the epidemic prevention channel, a set of return air circulation, ventilation, and purification system is mainly used to circulate and purify the internal air in the closed channel. Next, side-to-side blowing or a combination of side-to-side blowing and top-down blowing is used to shower and purify the surface of the personnel. If an infected person enters the channel, after the return air circulates, the virus exhaled by the infected person may continue to exist in the channel, making the personnel who enter the channel later face the risk of cross-infection. As a result, the epidemic spreads inside the building and leads to serious safety hazards.

Therefore, in view of the shortcomings of the abovementioned epidemic prevention channel, a research on an epidemic prevention method for an epidemic prevention channel to avoid the risk of cross-infection is an important issue.

Document KR 2012 0113324A describes a movable disinfection and sterilization apparatus sterilizing contagious viruses using an antiseptic solution and ultraviolet rays.

### SUMMARY

The Invention aims to provide an epidemic prevention channel to realize sterilization and to avoid the risk of cross infection at the same time.

The invention provides an epidemic prevention channel as defined in claim 1, including a channel body, wherein the channel body 1 is provided with an air inlet system, an air return system, and an exhaust system. An interior of the channel body is divided into an upper area and a lower area. The air inlet system includes air inlets, an air intake fan, a first filtering and sterilizing device, and a first nozzle group formed by a plurality of nozzles installed on a top wall of the channel body and side walls of the upper area. The air intake fan introduces external fresh air through the air inlets, the external fresh air passes through the air inlets, the air intake fan, the first filtering and sterilizing device, and the first nozzle group in sequence and is then sprayed into and purifies the upper area of the channel body. The air return system includes an air return port, an air return fan, a second filtering and sterilizing device, and a second nozzle group formed by a plurality of nozzles installed on side walls of the lower area of the channel body. The air in the channel body passes through the air return port, the air return fan, the second filtering and sterilizing device, and the second nozzle group in sequence and circulates, is sprayed into, and purifies the lower area of the channel body. The exhaust system includes an exhaust air inlet provided at a bottom of two ends of the channel body, an exhaust pipe communicating with the exhaust air inlet, an exhaust fan, a third filtering and sterilizing device, and an exhaust air outlet. The air discharged from the interior of the channel body passes through the exhaust air inlet, the exhaust pipe, the exhaust fan, the third filtering and sterilizing device, and the exhaust air outlet in sequence and flows to the outside of the epidemic prevention channel.

The epidemic prevention channel provided by the invention may be set at the entrance of places where people gather, such as stations, airports, shopping malls, and communities, to allow the people entering these places to be purified and sterilized. After a person enters the epidemic prevention channel, the external fresh air is pressurized by the air intake fan first, is sterilized by the first filtering and sterilizing device next, and is finally sprayed into the upper area of the channel body through the first nozzle group. Herein, the nozzles on the top wall of the channel body spray external fresh air from top to bottom, and the nozzles on the side walls of the upper area spray external fresh air oppositely. The air inlet system can quickly remove viruses, bacteria, and dust in the upper area, and can sterilize all parts of a person's body in the upper area through blowing. The air return system is used to guide the airflow direction in the channel body, so that the air in the channel body is pressurized by the air return fan, sterilized by the second filtering and sterilizing device, and then sprayed into the lower area of the channel body through the second nozzle group. The nozzles on the side walls of the lower area spray the airflow oppositely, and through blowing, all parts of the body in the lower area are fully sprayed and purified. The exhaust system is used to extract the air in the channel body, so as to control the pressure difference inside and outside the epidemic prevention channel together with the air inlet system. The extracted air is discharged from the exhaust air outlet after being processed by the third filtering and sterilizing device, such that secondary pollution caused by the exhausted air is prevented from being generated. The air inlet system, the air return system, and the exhaust system provided by the invention act simultaneously, so that the air in the channel body flows in an organized and directional manner. The upper area is continuously fed with filtered and sterilized external fresh air. Therefore, the air in the upper area continues to flow to the lower area, and the continuous high-speed airflow generates positive pressure so that the air in the lower area does not flow upwards, ensuring that the air inhaled by the person in the upper area is fresh and non-toxic air.

The epidemic prevention channel provided by the invention provides the following optimization improvements.

In the invention, hollow sandwich wall layers may be provided between the top wall and side walls of the channel body, and the sandwich wall layers include an upper layer and a lower layer. The upper layer and the lower layer are partitioned such that air cannot circulate. A top portion of the upper layer communicates with an outlet of the air intake fan, the upper layer also communicates with the first nozzle group, and the first filtering and sterilizing device is arranged in the upper layer. A bottom portion of the lower layer communicates with an outlet of the air return fan, the lower layer also communicates with the second nozzle group, and the second filtering and sterilizing device is arranged in the lower layer. The upper layer is provided with an air duct configured to communicate with the lower layers of two sandwich wall layers on the opposite side walls.

In the invention, top of the channel body may be provided with an air inlet box body, the air inlets are arranged at both ends of the air inlet box body, and the air intake fan is located in the air inlet box body.

In the invention, the air return port may be located on a side wall inside the channel body, and the air return port communicates with the lower layer of the sandwich wall layer through the air return fan.

In the invention, an exhaust box body may be arranged at an edge of the bottom of two ends of the channel body, the exhaust air inlet is arranged at the exhaust box body, the exhaust box body communicates with the exhaust pipe, and the exhaust pipe is connected to the exhaust fan.

In the invention, each of the first filtering and sterilizing device, the second filtering and sterilizing device, and the third filtering and sterilizing device may be one or a combination of any two filtering and sterilizing devices such as a primary filter, a high-efficiency filter, a high-pressure sterilization filter, and an ultraviolet sterilizer, and is configured for air sterilization, disinfection, and purification.

The invention further aims to provide an epidemic prevention method defined in claim 7, using the epidemic prevention channel. Each nozzle of the first nozzle group and the second nozzle group is nozzle with adjustable spraying direction. The air inlet system, the air return system, and the exhaust system control the pressure difference between the air inside and outside the channel body and control the flowing speed and direction of the airflow in the channel body to form the upper and lower air areas. The first nozzle group of the air inlet system feeds the external fresh air processed by the first filtering and sterilizing device into the upper area of the channel body, so that the air is sprayed into and purifies the upper area. The second nozzle group of the air return system circulates and feeds the air processed by the second filtering and sterilizing device into the lower area of the channel body, so that the air is sprayed into and purifies the lower area. The person entering the channel body is sprayed and sterilized. The first nozzle group continuously blows the air in the upper area to the lower area to ensure that the air inhaled by the person is the external fresh air processed by the first filtering and sterilizing device in the upper area.

Through the epidemic prevention method provided by the invention, it is ensured that the non-toxic air in the channel body is filtered and sterilized, especially the air in the upper area for inhalation is continuously updated to fresh non-toxic air to avoid cross-infection.

In the invention, the height of the epidemic prevention channel may be determined according to the height of the user. When the epidemic prevention channel is to be used by adults, the internal height of the channel body is 1.9 meters, and when the epidemic prevention channel is to be used by children, the height of the channel body is 1.6 meters. As for other usage scenarios, the height may be adjusted according to needs. The height of the upper area ≥ 2/5 of the height of the interior of the channel body, such that the body part above the armpit of the person inside the channel body is allowed to be located in the upper area.

In view of the foregoing, the invention features the following advantages.
(1) In the invention, through the air inlet system, the air return system, and the exhaust system, the interior of the epidemic prevention channel is divided into two air areas namely the upper area and the lower area. The airflow in the upper area continues to flow downwards, and the positive pressure created by the airflow from the upper area causes the return air circulation to only take place in the lower area, ensuring that no exhaled air remains in the upper area. The upper area provides fresh, non-toxic air introduced from the outside and filtered and sterilized for inhalation by personnel. Air is sprayed into and purifies the upper area through the air inlet system. Part of the air then circulates the channel and is sprayed into and purifies the lower area through the air return system. The air in the epidemic prevention channel flows out in an organized and directional manner through the exhaust system. In this way, the air breathed by each person entering the channel is fresh air that is filtered and sterilized. Therefore, cross-infection may not occur, and the purpose of epidemic prevention is achieved.
(2) In this exhaust system provided by the invention, the air discharged from the epidemic prevention channel is sterilized, and secondary pollution is thereby prevented from being generated.

### BRIEF DESCRIPTION OF THE DRAWINGS

FIG. 1 is a schematic view of an internal structure of an epidemic prevention channel according to the invention.
FIG. 2 is a schematic view of the movement of airflow in the epidemic prevention channel according to the invention.

### Description of Reference Numerals:

1: channel body, 2: air inlet, 3: air intake fan, 4: first filtering and sterilizing device, 5: first nozzle group, 6: air return port, 7: air return fan, 8: second filtering and sterilizing device, 9: air duct, 10: second nozzle group, 11: exhaust air inlet, 12: exhaust pipe, 13: exhaust fan, 14: third filtering and sterilizing device, 15: exhaust air outlet: 101: upper area, and 102: lower area.

### DESCRIPTION OF THE EMBODIMENTS

The technical solutions of the disclosure are further described in detail in combination with the accompanying drawings and embodiments.

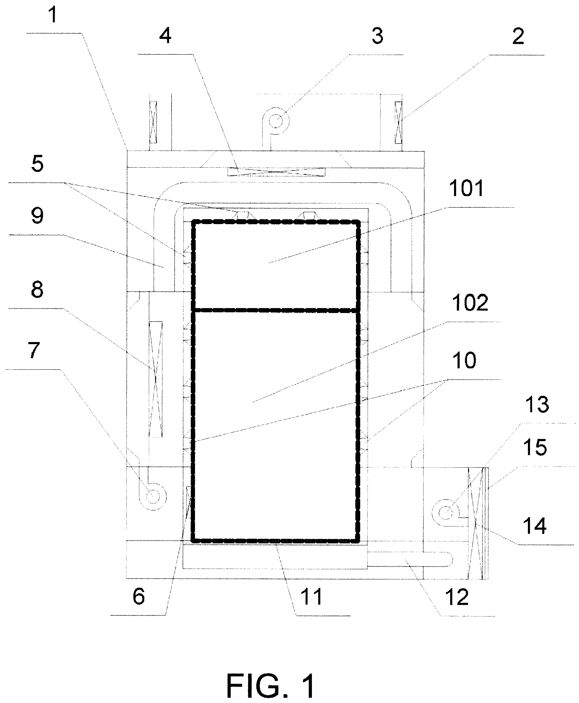

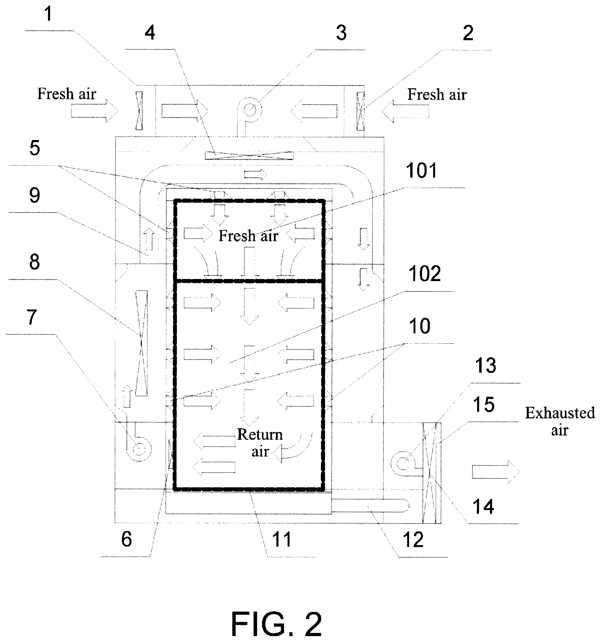

As shown in FIG. 1 and FIG. 2, the invention provides an epidemic prevention channel including a channel body 1, and the channel body 1 is provided with an air inlet system, an air return system, and an exhaust system. An interior of the channel body 1 is divided into an upper area 101 and a lower area 102. The air inlet system includes air inlets 2, an air intake fan 3, a first filtering and sterilizing device 4, and a first nozzle group 5 formed by a plurality of nozzles installed on a top wall of the channel body 1 and side walls of the upper area 101. The air intake fan 3 introduces external fresh air through the air inlets 2, the external fresh air passes through the air inlets 2, the air intake fan 3, the first filtering and sterilizing device 4, and the first nozzle group 5 in sequence and is then sprayed into and purifies the upper area 101 of the channel body 1. The air return system includes an air return port 6, an air return fan 7, a second filtering and sterilizing device 8, and a second nozzle group 10 formed by a plurality of nozzles installed on side walls of the lower area 102 of the channel body 1. The air in the channel body 1 passes through the air return port 6, the air return fan 7, the second filtering and sterilizing device 8, and the second nozzle group 10 in sequence and then circulates, is sprayed into, and purifies the lower area 102 of the channel body 1. The exhaust system includes an exhaust air inlet 11 provided at a bottom of two ends of the channel body 1, an exhaust pipe 12 communicating with the exhaust air inlet 11, an exhaust fan 13, a third filtering and sterilizing device 14, and an exhaust air outlet 15. The air in the interior of the channel body 1 may pass through the exhaust air inlet 11, the exhaust pipe 12, the exhaust fan 13, the third filtering and sterilizing device 14, and the exhaust air outlet 15 in sequence and flows to the outside of the epidemic prevention channel. The exhausted air is filtered and sterilized air and thus may not lead to secondary pollution.

In a preferred embodiment, the top of the channel body 1 is provided with an air inlet box body, the air inlets 2 are arranged at both ends of the air inlet box body, and the air intake fan 3 is located in the air inlet box body. Hollow sandwich wall layers are provided between the top wall and side walls of the channel body 1, the sandwich wall layers include an upper layer and a lower layer. The upper layer and the lower layer are partitioned such that air cannot circulate. A top portion of the upper layer communicates with an outlet of the air intake fan 3, the upper layer also communicates with the first nozzle group 5, and the first filtering and sterilizing device 4 is arranged in the upper layer. The first filtering and sterilizing device 4 is connected to the outlet of the air intake fan 3. The external fresh air is pressurized by the air intake fan 3, is sterilized by the first filtering and sterilizing device 4, and then flows into the upper layer, and the clean air flow is sent into the upper area 101 at a high speed through the first nozzle group 5 communicating with the upper layer.

Preferably, the air return port 6 is located on a side wall inside the channel body 1, and the air return port 6 communicates with the lower layer of the sandwich wall layer through the air return fan 7. A bottom portion of the lower layer communicates with an outlet of the air return fan 7, the lower layer also communicates with the second nozzle group 10, and the second filtering and sterilizing device 8 is arranged in the lower layer. The upper layer may be provided with an air duct 9 configured to communicate with the lower layers of two sandwich wall layers on the opposite side walls. The second filtering and sterilizing device 8 may be connected to the outlet of the air return fan 7. Part of the air processed by the second filtering and sterilizing device 8 is directly sprayed into the lower area 102 through the nozzles of the side wall where it is located, and the other part flows into the lower layer of the sandwich wall layer of the other side wall through the air duct 9 and is sprayed to the lower area 102 by the nozzles installed on the side wall, forming the effect of high-speed air-to-air blowing of clean air in the lower area 102.

Both the air intake fan 3 and the air return fan 7 are turned on. The nozzles in the first nozzle group 5 installed on the top wall spray from top to bottom, and the nozzles installed on the side wall of the upper area spray from opposite sides. The airflow in the upper area 101 continues to flow downwards, and the positive pressure created by the airflow from the upper area causes the return air circulation to only take place in the lower area.

An exhaust box body may be arranged at an edge of the bottom of two ends of the channel body 1, the exhaust air inlet 11 is arranged at the exhaust box body, the exhaust box body communicates with the exhaust pipe 12, and the exhaust pipe 12 is connected to the exhaust fan 13. While the air inlet system inputs fresh air into the channel body 1, the exhaust fan 13 leads the air to the outside of the epidemic prevention channel. By adjusting the pressure difference between the inside and outside of the channel body 1, it is ensured that fresh air is always introduced into the upper area 101 of the channel body 1.

In this embodiment, each of the first filtering and sterilizing device 4, the second filtering and sterilizing device 8, and the third filtering and sterilizing device 14 is one or a combination of any two filtering and sterilizing devices such as a primary filter, a high-efficiency filter, a high-pressure sterilization filter, and an ultraviolet sterilizer, and is configured for air sterilization, disinfection, and purification.

The invention further provides an epidemic prevention method as defined in claim 7, which is implemented through the following steps.

Each nozzle of the first nozzle group 5 and the second nozzle group 10 is nozzle with adjustable spray direction, so that the direction of the ejected airflow may be adjusted freely according to the height of a person or the key purification area to ensure good disinfection, sterilization, and purification effects. After a person enters the epidemic prevention channel, the air inlet system, the air return system, and the exhaust system control the pressure difference between the air inside and outside the channel body 1 and control the flowing speed and direction of the airflow in the channel body 1 to form the upper and lower air areas. The first nozzle group 1 of the air inlet system feeds the external fresh air processed by the first filtering and sterilizing device 4 into the upper area 101 of the channel body 1, so that the air is sprayed into and purifies the upper area 101 and sterilizes the various body parts of the person at the upper area 101. The second nozzle group 10 of the air return system circulates and feeds the air processed by the second filtering and sterilizing device 8 into the lower area 102 of the channel body 1, so that the air is sprayed into and purifies the lower area 102 and sterilizes the various body parts of the person at the lower area 102. The person entering the channel body 1 is sprayed and sterilized in this way. The first nozzle group 5 continuously blows the air in the upper area 101 to the lower area 102 to ensure that the air inhaled by the person is the external fresh air processed by the first filtering and sterilizing device 4 in the upper area 101.

In the invention, the nozzles on the side walls are arranged opposite to each other to ensure the air-to-air blowing effect.

The height of the epidemic prevention channel is determined according to the height of the user. For example, when the epidemic prevention channel is to be used by adults, the internal height of the channel body is 1.9 meters, and when the epidemic prevention channel is to be used by children, the height of the channel body is 1.6 meters. As for other usage scenarios, the height may be adjusted according to needs. The height of the upper area 101 may be ≥ 2/5 of the height of the interior of the channel body 1, such that the body part above the armpit of the person inside the channel body 1 is allowed to be located in the upper area 101.

The above embodiments do not limit the protection scope of the invention, and the embodiments of the invention are not limited thereto. The protection scope of the invention is defined in the appended claims.

## Claims

1. An epidemic prevention channel, **characterized in that**: comprising a channel body (1), the channel body (1) is provided with an air inlet system, an air return system, and an exhaust system, an interior of the channel body (1) is divided into an upper area (101) and a lower area (102), the air inlet system comprises air inlets (2), an air intake fan (3), a first filtering and sterilizing device (4), and a first nozzle group (5) formed by a plurality of nozzles installed on a top wall of the channel body (1) and side walls of the upper area (101), the air intake fan (3) introduces external fresh air through the air inlets (2), the external fresh air passes through the air inlets (2), the air intake fan (3), the first filtering and sterilizing device (4), and the first nozzle group (5) in sequence and is sprayed into and purifies the upper area (101) of the channel body (1), the air return system comprises an air return port (6), an air return fan (7), a second filtering and sterilizing device (8), and a second nozzle group (10) formed by a plurality of nozzles installed on side walls of the lower area (102) of the channel body (1) and, an air in the channel body (1) passes through the air return port (6), the air return fan (7), the second filtering and sterilizing device (8), and the second nozzle group (10) in sequence and circulates, is sprayed into, and purifies the lower area (102) of the channel body (1), the exhaust system comprises an exhaust air inlet (11) provided at a bottom of two ends of the channel body (1), an exhaust pipe (12) communicating with the exhaust air inlet (11), an exhaust fan (13), a third filtering and sterilizing device (14), and an exhaust air outlet (15), and the air discharged from the interior of the channel body (1) passes through the exhaust air inlet (11), the exhaust pipe (12), the exhaust fan (13), the third filtering and sterilizing device (14), and the exhaust air outlet (15) in sequence and flows to the outside of the epidemic prevention channel.

2. The epidemic prevention channel according to claim 1, **characterized in that**: hollow sandwich wall layers are provided between the top wall and side walls of the channel body (1), the sandwich wall layers comprise an upper layer and a lower layer, the upper layer and the lower layer are partitioned such that air cannot circulate, a top portion of the upper layer communicates with an outlet of the air intake fan (3), the upper layer also communicates with the first nozzle group (5), the first filtering and sterilizing device (4) is arranged in the upper layer, a bottom portion of the lower layer communicates with an outlet of the air return fan (7), the lower layer also communicates with the second nozzle group (10), the second filtering and sterilizing device (8) is arranged in the lower layer, and the upper layer is provided with an air duct (9) configured to communicate with the lower layers of two sandwich wall layers on the opposite side walls.

3. The epidemic prevention channel according to claim 2, **characterized in that**: the top of the channel body (1) is provided with an air inlet box body, the air inlets (2) are arranged at both ends of the air inlet box body, and the air intake fan (3) is located in the air inlet box body.

4. The epidemic prevention channel according to claim 3, **characterized in that**: the air return port (6) is located on a side wall inside the channel body (1), and the air return port (6) communicates with the lower layer of the sandwich wall layers through the air return fan (7).

5. The epidemic prevention channel according to claim 4, **characterized in that**: an exhaust box body is arranged at an edge of the bottom of the two ends of the channel body (1), the exhaust air inlet (11) is arranged at the exhaust box body, the exhaust box body communicates with the exhaust pipe (12), and the exhaust pipe (12) is connected to the exhaust fan (13).

6. The epidemic prevention channel according to claim 5, **characterized in that**: each of the first filtering and sterilizing device (4), the second filtering and sterilizing device (8), and the third filtering and sterilizing device (14) is one or a combination of any two filtering and sterilizing devices such as a primary filter, a high-efficiency filter, a high-pressure sterilization filter, and an ultraviolet sterilizer, and is configured for air sterilization, disinfection, and purification.

7. An epidemic prevention method using the epidemic prevention channel according to any one of claims 1 to 6, **characterized in that**: each nozzle of the first nozzle group (5) and the second nozzle group (10) is nozzle with adjustable spraying direction, controlling, through the air inlet system, the air return system, and the exhaust system, a pressure difference between the air inside and outside the channel body and controlling a flowing speed and direction of an airflow in the channel body to form the upper and lower air areas; feeding, through the first nozzle group (5) of the air inlet system, the external fresh air processed by the first filtering and sterilizing device (4) into the upper area (101) of the channel body (1), circulating and feeding, through the second nozzle group (10) of the air return system, the air processed by the second filtering and sterilizing device (8) into the lower area (102) of the channel body (1), spraying and sterilizing a personnel entering the channel body (1); and continuously blowing, by the first nozzle group (5), the air in the upper area (101) to the lower area (102) to ensure that an air inhaled by a person is the external fresh air processed by the first filtering and sterilizing device (4) in the upper area (101).

8. The epidemic prevention method according to claim 7, **characterized in that**: a height of the upper area (101) is ≥ 2/5 of a height of the interior of the channel body (1), such that a body part above an armpit of the person inside the channel body (1) is allowed to be located in the upper area (101).

## Patentansprüche

1. Epidemieverhinderungskanal, **gekennzeichnet durch**: umfassend einen Kanalkörper (1), der Kanalkörper (1) mit einem Lufteinlasssystem, einem Luftrückführungssystem und einem Auslasssystem bereitgestellt ist, ein Innenraum des Kanalkörpers (1) in einen oberen Bereich (101) und einen unteren Bereich (102) unterteilt ist, das Lufteinlasssystem Lufteinlässe (2), ein Lufteinlassgebläse (3), eine erste Filter- und Sterilisationsvorrichtung (4) und eine erste Düsengruppe (5) umfasst, die aus einer Vielzahl von Düsen gebildet ist, die an einer oberen Wand des Kanalkörpers (1) und an Seitenwänden des oberen Bereichs (101) installiert sind, das Lufteinlassgebläse (3) externe Frischluft durch die Lufteinlässe (2) einführt, die externe Frischluft durch die Lufteinlässe (2), das Lufteinlassgebläse (3), die erste Filter- und Sterilisationsvorrichtung (4) und die erste Düsengruppe (5) nacheinander durchläuft und in den oberen Bereich (101) des Kanalkörpers (1) gesprüht wird und diesen reinigt, das Luftrückführungssystem eine Luftrückführungsöffnung (6), ein Luftrückführgebläse (7), eine zweite Filter- und Sterilisationsvorrichtung (8) und eine zweite Düsengruppe (10) umfasst, die durch eine Vielzahl von Düsen gebildet wird, die an den Seitenwänden des unteren Bereichs (102) des Kanalkörpers (1) installiert sind, und wobei eine Luft in dem Kanalkörper (1) durch die Luftrückführöffnung (6), das Luftrückführgebläse (7), die zweite Filter- und Sterilisationsvorrichtung (8) und die zweite Düsengruppe (10) nacheinander durchläuft und zirkuliert, wird in den unteren Bereich (102) des Kanalkörpers (1) gesprüht und reinigt, wobei das Abluftsystem einen Ablufteinlass (11) umfasst, der an einem Boden von zwei Enden des Kanalkörpers (1) bereitgestellt ist, ein Abluftrohr (12), das mit dem Ablufteinlass (11) in Verbindung steht, ein Abluftgebläse (13), eine dritte Filter- und Sterilisationsvorrichtung (14) und einen Abluftauslass (15), und die aus dem Inneren des Kanalkörpers (1) abgegebene Luft strömt nacheinander durch den Ablufteinlass (11), das Abluftrohr (12), das Abluftgebläse (13), die dritte Filter- und Sterilisationsvorrichtung (14) und den Abluftauslass (15) und zu der Außenseite des Epidemieverhinderungskanals strömt.

2. Epidemieverhinderungskanal gemäß Anspruch 1, **dadurch gekennzeichnet, dass**: hohle Sandwich-Wandschichten zwischen der oberen Wand und den Seitenwänden des Kanalkörpers (1) bereitgestellt sind, die Sandwich-Wandschichten eine obere Schicht und eine untere Schicht umfassen, die obere Schicht und die untere Schicht so unterteilt sind, dass keine Luft zirkulieren kann, ein oberer Abschnitt der oberen Schicht mit einem Auslass des Lufteinlassgebläses (3) in Verbindung steht, die obere Schicht auch mit der ersten Düsengruppe (5) in Verbindung steht, die erste Filter- und Sterilisationsvorrichtung (4) in der oberen Schicht angeordnet ist, ein unterer Abschnitt der unteren Schicht mit einem Auslass des Luftrückführgebläses (7) in Verbindung steht, die untere Schicht auch mit der zweiten Düsengruppe (10) in Verbindung steht, die zweite Filter- und Sterilisationsvorrichtung (8) in der unteren Schicht angeordnet ist und die obere Schicht mit einem Luftkanal (9) bereitgestellt ist, der konfiguriert ist, um mit den unteren Schichten von zwei Sandwich-Wandschichten an den gegenüberliegenden Seitenwänden in Verbindung steht.

3. Epidemieverhinderungskanal gemäß Anspruch 2, **dadurch gekennzeichnet, dass**: die Spitze des Kanalkörpers (1) mit einem Lufteinlasskastenkörper bereitgestellt ist, die Lufteinlässe (2) an beiden Enden des Lufteinlasskastenkörpers angeordnet sind und das Lufteinlassgebläse (3) sich in dem Lufteinlasskastenkörper befindet.

4. Epidemieverhinderungskanal gemäß Anspruch 3, **dadurch gekennzeichnet, dass** die Luftrückführöffnung (6) sich an einer Seitenwand im Inneren des Kanalkörpers (1) befindet und die Luftrückführöffnung (6) mit der unteren Schicht der Sandwich-Wandschichten über das Luftrückführgebläse (7) in Verbindung steht.

5. Epidemieverhinderungskanal gemäß Anspruch 4, **dadurch gekennzeichnet, dass**: ein Abluftkastenkörper an einem Rand des Bodens der beiden Enden des Kanalkörpers (1) angeordnet ist, der Ablufteinlass (11) an dem Abluftkastenkörper angeordnet ist, der Abluftkastenkörper mit dem Abluftrohr (12) in Verbindung steht und das Abluftrohr (12) mit dem Abluftventilator (13) verbunden ist.

6. Epidemieverhinderungskanal gemäß Anspruch 5, **dadurch gekennzeichnet, dass**: jede der ersten Filter- und Sterilisationsvorrichtung (4), der zweiten Filter- und Sterilisationsvorrichtung (8) und der dritten Filter- und Sterilisationsvorrichtung (14) eine oder eine Kombination von zwei beliebigen Filter- und Sterilisationsvorrichtungen ist, wie beispielsweise ein Primärfilter, ein Hochleistungsfilter, ein Hochdruck-Sterilisationsfilter und ein Ultraviolett-Sterilisator, und zur Luftsterilisation, Desinfektion und Reinigung konfiguriert ist.

7. Epidemieverhinderungsverfahren unter Verwendung des Epidemieverhinderungskanal gemäß einem der Ansprüche 1 bis 6, **dadurch gekennzeichnet, dass**: jede Düse der ersten Düsengruppe (5) und der zweiten Düsengruppe (10) eine Düse mit einstellbarer Sprührichtung ist, die über das Lufteinlasssystem, das Luftrückführungssystem und das Abluftsystem eine Druckdifferenz zwischen der Luft innerhalb und außerhalb des Kanalkörpers steuert und eine Strömungsgeschwindigkeit und -richtung eines Luftstroms in dem Kanalkörper steuert, um die oberen und unteren Luftbereiche zu bilden; Zuführen der von der ersten Filter- und Sterilisationsvorrichtung (4) aufbereiteten externen Frischluft durch die erste Düsengruppe (5) des Lufteinlasssystems in den oberen Bereich (101) des Kanalkörpers (1), Zirkulieren und Zuführen der von der zweiten Filter- und Sterilisationsvorrichtung (8) aufbereiteten Luft durch die zweite Düsengruppe (10) des Luftrückführungssystems in den unteren Bereich (102) des Kanalkörpers (1), Besprühen und Sterilisieren einer in den Kanalkörper (1) eintretenden Person; und kontinuierliches Blasen der Luft in dem oberen Bereich (101) in den unteren Bereich (102) durch die erste Düsengruppe (5), um sicherzustellen, dass die von einer Person eingeatmete Luft die von der ersten Filter- und Sterilisationsvorrichtung (4) in dem oberen Bereich (101) aufbereitete Außenfrischluft ist.

8. Epidemieverhinderungsverfahren gemäß Anspruch 7, **dadurch gekennzeichnet, dass**: eine Höhe des oberen Bereichs (101) ≥ 2/5 einer Höhe des Inneren des Kanalkörpers (1) ist, so dass ein Körperteil oberhalb einer Achselhöhle der Person sich in dem Inneren des Kanalkörpers (1) in dem oberen Bereich (101) befinden kann.

## Revendications

1. Canal de prévention des épidémies, **caractérisé en ce que** : il comprend un corps de canal (1), le corps de canal (1) est pourvu d'un système d'entrée d'air, un système de retour d'air et un système d'évacuation, un intérieur du corps de canal (1) est divisé en une zone supérieure (101) et une zone inférieure (102), le système d'entrée d'air comprend des entrées d'air (2), un ventilateur d'admission d'air (3),un premier dispositif de filtrage et de stérilisation (4) et un premier groupe de buses (5) formé par une pluralité de buses installées sur une paroi de sommet du corps de canal (1) et des parois latérales de la zone supérieure (101), le ventilateur d'admission d'air (3) introduit de l'air frais externe à travers les entrées d'air (2), l'air frais externe traverse les entrées d'air (2), le ventilateur d'admission d'air (3), le premier dispositif de filtrage et de stérilisation (4) et le premier groupe de buses (5) successivement et est pulvérisé dans et purifie la zone supérieure (101) du corps de canal (1), le système de retour d'air comprend un orifice de retour d'air (6), un ventilateur de retour d'air (7), un deuxième dispositif de filtrage et de stérilisation (8) et un deuxième groupe de buses (10) formé par une pluralité de buses installées sur des parois latérales de la zone inférieure (102) du corps de canal (1) et un air dans le corps de canal (1) traverse l'orifice de retour d'air (5), le ventilateur de retour d'air (7), le deuxième dispositif de filtrage et de stérilisation (8) et le deuxième groupe de buses (10) successivement et circule, est pulvérisé dans et purifie la zone inférieure (102) du corps de canal (1), le système d'évacuation comprend une entrée d'air d'évacuation (11) prévue au niveau d'une base des deux extrémités du corps de canal (1), un tuyau d'évacuation (12) communiquant avec l'entrée d'air d'évacuation (11), un ventilateur d'évacuation (13), un troisième dispositif de filtrage et de stérilisation (14) et une sortie d'air d'évacuation (15) et l'air évacué de l'intérieur du corps de canal (1) traverse l'entrée d'air d'évacuation (11), le tuyau d'évacuation (12), le ventilateur d'évacuation (13), le troisième dispositif de filtrage et de stérilisation (14) et la sortie d'air d'évacuation (15) successivement et circule vers l'extérieur du canal de prévention des épidémies.

2. Canal de prévention des épidémies selon la revendication 1, **caractérisé en ce que** : des couches de parois intermédiaires creuses sont prévues entre la paroi de sommet et les parois latérales du corps de canal (1), les couches de parois intermédiaires comprennent une couche supérieure et une couche inférieure, la couche supérieure et la couche inférieure sont partitionnées de sorte que l'air ne puisse pas circuler, une partie de sommet de la couche supérieure communique avec une sortie du ventilateur d'admission d'air (3), la couche supérieure communique aussi avec le premier groupe de buses (5), le premier dispositif de filtrage et de stérilisation (4) est disposé dans la couche supérieure, une partie de base de la couche inférieure communique avec une sortie du ventilateur de retour d'air (7), la couche inférieure communique aussi avec le deuxième groupe de buses (10), le deuxième dispositif de filtrage et de stérilisation (8) est disposé dans la couche inférieure et la couche supérieure est pourvue d'une conduite d'air (9) configurée pour communiquer avec les couches inférieures de deux couches de parois intermédiaires sur les parois latérales opposées.

3. Canal de prévention des épidémies selon la revendication 2, **caractérisé en ce que** : le sommet du corps de canal (1) est pourvu d'un corps de boîte d'admission d'air, les entrées d'air (2) sont disposées aux deux extrémités du corps de boîte d'admission d'air et le ventilateur d'admission d'air (3) est situé dans le corps de boîte d'admission d'air.

4. Canal de prévention des épidémies selon la revendication 3, **caractérisé en ce que** : le port de retour d'air (6) est situé sur une paroi latérale à l'intérieur du corps de canal (1) et l'orifice de retour d'air (6) communique avec la couche inférieure des couches de parois intermédiaires via le ventilateur de retour d'air (7).

5. Canal de prévention des épidémies selon la revendication 4, **caractérisé en ce que** : un corps de boîte d'échappement est disposé au niveau d'un bord de la base des deux extrémités du corps de canal (1), l'entrée d'air d'échappement (11) est disposée au niveau du corps de boîte d'échappement, le corps de boîte d'échappement communique avec le tuyau d'évacuation (12) et le tuyau d'évacuation (12) est raccordé au ventilateur d'évacuation (13).

6. Canal de prévention des épidémies selon la revendication 5, **caractérisé en ce que** : chacun du premier dispositif de filtrage et de stérilisation (4), le deuxième dispositif de filtrage et de stérilisation (8) et le troisième dispositif de filtrage et de stérilisation (14) est un ou une combinaison de n'importe lesquels de deux dispositifs de filtrage et de stérilisation comme un filtre primaire, un filtre à haut rendement, un filtre de stérilisation à haute pression et un stérilisateur à ultraviolets et est configuré pour la stérilisation, désinfection et purification d'air.

7. Procédé de prévention des épidémies utilisant le canal de prévention des épidémies selon une quelconque des revendications 1 à 6, **caractérisé en ce que** : chaque buse du premier groupe de buses (5) et du deuxième groupe de buses (10) est une buse avec une direction de pulvérisation réglable, régulant, via le système d'entrée d'air, le système de retour d'air et le système d'échappement, une différence de pression entre l'air à l'intérieur et à l'extérieur du corps de canal et régulant une vitesse et direction de circulation d'un flux d'air dans le corps de canal pour former les zones d'air supérieure et inférieure ; alimenter, via le premier groupe de buses (5) du système d'entrée d'air, l'air frais externe traité par le premier dispositif de filtrage et de stérilisation (4) dans la zone supérieure (101) du corps de canal (1), faire circuler et alimenter, via le deuxième groupe de buses (10)du système de retour d'air, l'air traité par le deuxième dispositif de filtrage et de stérilisation (8) dans la zone inférieure (102) du corps de canal (1),pulvériser et stériliser un personnel entrant dans le corps de canal (1) ; et souffler continuellement, par le premier groupe de buses (5), l'air dans la zone supérieure (101) vers la zone inférieure (102) pour garantir qu'un air inhalé par une personne est l'air frais externe traité par le premier dispositif de filtrage et de stérilisation (4) dans la zone supérieure (101).

8. Procédé de prévention des épidémies selon la revendication 7, **caractérisé en ce que** : une hauteur de la zone supérieure (101) est ≥ 2/5 d'une hauteur de l'intérieur du corps de canal (1), de sorte qu'un partie du corps au-dessus des aisselles de la personne à l'intérieur du corps de canal (1) est autorisée à être située dans la zone supérieure (101).
